# DEMANDE DE BREVET EUROPEEN

(11) **EP 1 158 055 A1**
(43) Date de publication de la demande: **28.11.2001**
(21) Numéro de dépôt: 00111370.3
(22) Date de dépôt: 26.05.2000
(51) Int. Cl.: C12Q 1/68, G01N 33/574

(54) **Méthode pour le diagnostic de cancers**

(71) Demandeur: Chen, Xu Qi, University of Teaxs, Laboratoire de Leucémie, Houston, Texas 77030 (US); Stroun, Maurice, 1208 Geneve (CH); Anker, Philippe, 1203 Geneve (CH)
(72) Inventeur: Chen, Xu Qi, University of Teaxs, Laboratoire de Leucémie, Houston, Texas 77030 (US); Stroun, Maurice, 1208 Geneve (CH); Anker, Philippe, 1203 Geneve (CH)
(74) Mandataire: Micheli & Cie

(57) **Abrégé**

L'invention concerne une méthode pour le diagnostic et/ou le suivi de l'évolution de cancers comprenant l'analyse des composants ARN de l'enzyme télomérase présents dans le plasma ou le sérum sanguin.

## Description

La présente invention concerne une méthode de diagnostic et/ou de suivi de l'évolution de divers types de cancers, par exemple après un traitement de chimiothérapie ou après une opération.

On sait que le diagnostic et le suivi de l'évolution des cancers sont effectués, à part l'observation et l'examen direct de tumeurs, par analyse de biopsies ou, dans le cas de cancers du sang, de la moelle osseuse, ce qui implique soit une intervention chirurgicale, soit un test invasif du type biopsie ou aspiration médullaire. Or, en plus du caractère désagréable, voire dangereux, pour les patients de telles méthodes, il a été constaté qu'elles pouvaient en outre être peu précises. En ce qui concerne le cancer du sein, de grands efforts sont faits pour développer le test de détection par mammographie. Bien que plusieurs études indiquent que la mammographie de masse peut être une stratégie utile pour réduire la mortalité due au cancer du sein, cette méthode comporte un certain nombre de désavantages. Parmi ceux-ci, il faut relever un fort taux de faux positifs, des faux négatifs fréquents et d'énormes dépenses pour l'Etat. Ainsi, lorsque les avantages et les désavantages sont comparés, il n'est pas surprenant que cette forme d'examen ait engendré des débats contradictoires animés depuis vingt ans.

Le but de cette invention consiste donc à fournir une méthode de diagnostic et/ou de suivi de l'évolution de divers types de cancers qui soit d'une part plus précise et plus fiable, et d'autre part qui soit plus facile à réaliser et n'impliquant pas de test invasif sur les patients.

La méthode de diagnostic et/ou suivi de l'évolution de cancers, objet de l'invention et visant à atteindre le but précité, comprend l'analyse du composant ribonucléique (ARN) de la ribonucléoprotéine télomérase dans le plasma ou le sérum sanguin.

La télomérase est une enzyme de nature ribonucléoprotéique qui synthétise des séquences répétées télomériques sur les terminaisons des chromosomes. Les télomères protègent les extrémités des chromosomes, et à chaque division cellulaire les télomères se raccourcissent. La télomérase utilise pour synthétiser ces télomères un ou deux segments de son composant ARN en tant que matrice.

L'activité de cette enzyme est devenue un indicateur établi pour le diagnostic et le pronostic de la plupart des tumeurs cancéreuses. L'expression de l'ARN de télomérase humaine (hTR) ou de l'enzyme de transcriptase inverse de l'ARN télomérase (hTERT) a été déterminée lors de la progression de plusieurs espèces de tumeurs, ce qui a permis d'établir une corrélation entre cette expression (la quantité d'ARN) et l'activité de la télomérase. La plupart des cancers et des lignées cellulaires immortalisées ont une forte activité télomérasique, reflet d'un mécanisme échappant aux régulations normales de vieillissement.

Or, et bien que les composants ARN de la télomérase soient des composants cellulaires, il a été constaté que, de manière surprenante, ces composants se retrouvaient également sous forme extra-cellulaire dans le plasma ou le sérum sanguin.

En effet, les présents inventeurs ont mis en évidence la présence de hTR et de hTERT dans le plasma ou le sérum sanguin de personnes souffrant notamment de cancers du sein, de l'ovaire, de l'estomac ou du colon, alors que ces produits sont absents dans le sang de personnes saines. Contrairement aux divers marqueurs d'acide désoxyribonucléique (ADN) déjà retrouvés dans le plasma ou le sérum, tels les mutations du gène ras ou p53 ou les nombreux microsatellites de la littérature, les composants ARN de la télomérase semblent pouvoir être employés pour toutes les sortes de tumeurs. Il s'agit d'un marqueur plasmatique ou séreux général pour le cancer. Ainsi pour la plupart des cancers, le taux de sérums porteurs de composants ARN de la télomérase est plus important que celui obtenu par les autres marqueurs d'acides nucléiques employés jusqu'à maintenant.

Plus particulièrement, la méthode de diagnostic selon l'invention consiste à extraire l'ARN du plasma ou du sérum sanguin, à purifier et à amplifier cet ARN, puis à établir la présence et optionnellement la quantité de produit de réaction polymérase en chaîne reverse (RT PCR) représentant les composants hTR ou hTERT, ceci de manière comparative entre le plasma ou le sérum d'une personne présumée malade et celui d'une personne en bonne santé.

Les produits d'amplification PCR des composants ARN transcrits en ADN grâce à la RT PCR sont mis en évidence et le cas échéant quantifiés par exemple en utilisant des méthodes à coloration sur gel. Cette analyse peut également se faire différemment sur d'autres gels ou sans gel par technique immunologique radioactive (RIA), par ELISA (Enzyme Linked Immunosorbent Assay ou par test microchips (gene array). Une amplification quantitative sur Taq Man (Perkin Elmer Biosystem) ou sur capillaires Light Cycler (Hofmann-La Roche) améliore la précision de la détection du hTR et du hTERT.

De même, toute technique d'extraction, de purification et d'amplification de l'ARN dans le plasma ou le sérum peut être employée.

La présente invention sera maintenant illustrée de manière non limitative par l'Exemple qui suit, relatif au diagnostic des cancers du sein. Toutefois, il convient de constater que la portée de la présente invention n'est en aucune manière limitée au diagnostic de ce type de cancer. Des tests préliminaires ont en effet démontré également la présence de hTR et hTERT dans le sérum de patients atteints notamment de cancers ovariens ou gastro-intestinaux. On peut en conclure d'ores et déjà que les hTR et hTERT du plasma ou du sérum sont à même de constituer des marqueurs généraux de nombreux types de cancers.

### Exemple

### Diagnostic du cancer du sein par détection de hTR ou de hTERT dans le plasma ou le sérum sanguin

Des échantillons de sang (2-3 ml) ont été prélevés préopérativement sur 18 patientes, porteuses de petites tumeurs cancéreuses du sein (T1 ou T2) encore différenciées (G1 ou G2) et en principe sans nodules cancéreux ni métastases. Les échantillons ont ensuite été centrifugés à 900 g pendant 15 minutes à température ambiante et le sérum recueilli. Une seconde centrifugation à 900g pendant 15 min. a été effectuée pour se débarrasser de tous débris. Les échantillons de sérum ont été conservés à -70°C.

L'extraction de l'ARN a été effectuée en utilisant une technique usuelle et un kit commercial ("SV Total RNA Isolation System" de la société Promega Wl, USA), selon les instructions du fournisseur. Par rapport à ces instructions une modification a été apportée, consistant plus particulièrement en l'adjonction de 175 µl de tampon "SVRNA Lysis" directement à 100µl de sérum ou de plasma frais (ou du moins n'ayant pas été dégélé plus d'une fois).

Détection de hTR, de hTERT et du rRNA (RNA de référence) au moyen de la PCR par transcriptase réverse (RT-PCR) :

Pour la détection de la présence et de la quantité de hTR et de rRNA, on a utilisé un kit Qiagen (One Step RT-PCR). 1 mg d'ARN de sérum a été introduit dans un mélange réactionnel de 25 µl RT-PCR contenant 400µM de chaque dNTP, omniscript ™ reverse transcriptase, sensiscript ™ reverse transcriptase, hot-start Taq™ DNA polymerase et 0,15µM d'amorce (primer) à savoir pour hTR (sens) GAAGGGCGTAGGCGCCGTGCTTTTGC et (antisens) GTTTGCTCTAGAATGAACGGTGGAAGG. Le mélange a été incubé à 50°C pendant au moins 30 min. pour la transcription reverse, puis à 95°C pendant 15 min. pour activer la "hot-start Taq ™ DNA Polymerase", et 50 cycles à 94°C pendant 30 sec., à 65°C pendant 1 min., à 72°C pendant 1 min., avec une extension finale à 72°C pendant 10 min.

Les mêmes conditions de RT-PCR ont été utilisées pour la détection de hTERT, avec toutefois une concentration d'amorce de 0,3 µM, les amorces étant les suivantes : (sens) TGACACCTCACCTCACCCAC et (antisens) CACTGTCTTCCGCAAGTTCAC.

En ce qui concerne le RNA de contrôle, le GAPDH et les mêmes conditions que pour hTR ont également été employées, avec une concentration d'amorce de 0,3 µM; les séquences de l'amorce GAPDH étant les suivantes : (sens) CGGAGTCAACGGATTTGGTCGTAT et (antisens) AGCCTTCTCCATGGTGGTGAAGAC.

Toutes les séquences d'acides aminés mentionnées ci-dessus à titre d'exemples d'amorces sont connues en tant que telles, et peuvent par exemple être consultées sur le site Internet de la Genome Database (http://www.gdb.org/).

La PCR a donné un produit d'amplification de 111bp pour le hTR, de 95 bp pour le hTERT et de 306 bp pour GAPDH du RNA de contrôle. Ce résultat a été analysé à 55°C sur gels "Elchrom Scientific S-50" (Elchrom Scientific, Cham, Suisse), coloré au "SYBR-gold" (Molecular Probe, Eugene, OR, USA) pendant 45 min., décoloré 2 fois pendant 30 min. dans de l'eau déionisée à température ambiante et dans l'obscurité.

### Résultats :

En mettant en oeuvre la technique décrite ci-dessus, il en résulte qu'il est effectivement possible de détecter un produit PCR issu de l'ARN de référence dans tous les échantillons analysés. Quantitativement, tous les échantillons sont semblables, qu'ils proviennent du sérum d'une personne saine ou de celui d'une personne malade.

En ce qui concerne le produit d'amplification obtenu avec les amorces spécifiques de l'hTR, seul l'ARN provenant de patientes souffrant d'un cancer du sein s'est révélé positif dans 25% des cas. Quant à l'autre composant ARN de l'enzyme, hTERT, il a été détecté dans 22% des sérums des patientes malades. La combinaison des deux marqueurs a donc permis de détecter un cancer du sein dans 45% des cas; ce taux est clairement supérieur à celui que l'on peut obtenir avec les techniques actuellement connues, lequel dépasse rarement environ 30%. Par contre, les deux composants ARN de la télomérase sont absents des sérums de contrôle (personnes saines).

## Revendications

1. Méthode pour le diagnostic et/ou le suivi de l'évolution de cancers comprenant l'analyse des composants ARN de l'enzyme télomérase présents dans le plasma ou le sérum sanguin.

2. Méthode selon la revendication 1, **caractérisée par le fait qu'**on extrait de l'ARN du plasma ou du sérum, qu'on purifie et qu'on amplifie cet ARN, puis qu'on analyse les composants ARN de la télomérase.

3. Méthode selon la revendication 2, **caractérisée par le fait que** l'ARN est amplifié par la technique de transcriptase reverse en chaîne (RT-PCR).

4. Méthode selon la revendication 3, **caractérisée par le fait que** les composants analysés sont hTR, hTERT et un RNA de référence correspondant à l'expression d'un gène codant.

5. Méthode selon la revendication 4, **caractérisée par le fait que** lesdits composants sont analysés par coloration sur gels, par technique immunologique radioactive (RIA), par test enzyme linked immounosorbent (ELISA) ou par test microchips (gene array), et éventuellement soumis à une quantification.

6. Méthode selon la revendication 5, **caractérisée par le fait que** la quantification des composants est effectuée sur "Taq Man" ou sur capillaires "Light Cycler".
